# NEW EUROPEAN PATENT SPECIFICATION

(11) **EP 0 839 039 B2**
(45) Date of publication and mention of the opposition decision: **06.06.2007**
(45) Mention of the grant of the patent: 05.11.2003
(21) Application number: 96926388.8
(22) Date of filing: 19.07.1996
(51) Int. Cl.: A61K 31/445, A61K 47/48, A61P 3/00, A61P 5/00, A61P 25/00, A61P 29/00

(54) **PAROXETINE CONTROLLED RELEASE COMPOSITIONS**
MITTEL ZUR GESTEUERTEN FREISETZUNG VON PAROXETIN
COMPOSITIONS DE PAROXETINE A LIBERATION CONTROLEE

(30) Priority: 20.07.1995 GB 9514842
(43) Date of publication of application: 06.05.1998
(60) Divisional application: 03078259.3
(73) Proprietor: SMITHKLINE BEECHAM PLC, Brentford, Middlesex TW8 9GS (GB)
(72) Inventor: LEONARD, Graham S., GlaxoSmithKline, Third Avenue, Harlow, Essex, CM19 5AW (GB); ELDER, David P., GlaxoSmithKline, Ware, Hertfordshire, SG12 0DP (GB)
(74) Representative: West, Vivien
(86) International application number: PCT/EP1996/003252
(87) International publication number: WO 1997/003670

(56) References cited:
- EP-A- 0 432 607
- WO-A-92/03124
- WO-A-95/15155
- CHEMICAL ABSTRACTS, vol. 124, no. 10, 4 March 1996 Columbus, Ohio, US; abstract no. 127144, XP002018196 & CA,A,2 143 070 (P.MODI) 23 August 1995
- Perucca et al.; Clin. Pharmacokinet, 27(3), pp. 175-190 (1994)
- Pharmaceutics, The Science of Dosage Form Design, pp. 204-211, edited by Michael E. Aulton (1988)

## Description

The present invention relates to a formulation containing paroxetine or a pharmaceutically acceptable salt thereof, and to its use in the treatment and/or prophylaxis of certain disorders.

US Patent No 4,007,196 describes ***inter alia*** a compound which is commonly known as paroxetine. This compound is a Selective Serotonin Reuptake Inhibitor (SSRI) and is currently marketed world-wide for the treatment and/or prophylaxis of depression.

The current formulation which is the only marketed formulation of paroxetine hydrochloride is a swallow tablet.

It has now been surprisingly found that controlled release and delayed release formulations containing paroxetine give rise to an unexpected reduction in the side effects associated with swallow tablets.

Accordingly, the present invention provides a controlled release and delayed release formulation which is adapted or intended for oral administration and which contains paroxetine or a pharmaceutically acceptable salt thereof.

By controlled release is meant any formulation technique wherein release of the active substance from the dosage from is modified to occur at a slower rater than that from an immediate release product, such as a conventional swallow tablet or capsule.

By delayed release is meant any formulation technique wherein release of the active substance from the dosage form is modified to occur at a later time than that from a conventional immediate release product. The subsequent release of active substance from a delayed release formulation may also be controlled as defined above.

Examples of controlled release formulations which are suitable for incorporating paroxetine are described in:
Sustained Release Medications, Chemical Technology Review No. 177. Ed. J.C. Johnson. Noyes Data Corporation 1980.
Controlled Drug Delivery, Fundamentals and Applications, 2nd Edition. Eds. J.R. Robinson, V.H.L. Lee. Mercel Dekkes Inc. New York 1987.

Examples of delayed release formulations which are suitable for incorporating paroxetine are described in:
Remington's Pharmaceutical Sciences 16th Edition, Mack Publishing Company 1980, Ed. A. Osol.

Such controlled release formulations are preferably formulated in a manner such that release of paroxetine is effected predominantly during the passage through the stomach and the small intestine, and delayed release formulations are preferably formulated such that release of paroxetine is avoided in the stomach and is effected predominantly during passage through the small intestine

Said formulations are preferably formulated such that the release of the active substance is predominantly 1½ to 3 hours post ingestion.

The small intestine is suitably the duodenum, the ileum or the jejunem.

Patients who benefit most from the formulations of the present invention are those who are known to suffer from nausea upon oral administration using swallow tablets.

Preferred formulations are ultimately enteric coated tablets or caplets, wax or polymer coated tablets or caplets or time-release matrices, or combinations thereof.

Particularly preferred formulations are described in US Patent No. 5,102,666.

Preferably a formulation of the invention comprises a polymeric controlled release composition comprising a reaction complex formed by the interaction of (1) a calcium polycarbophil component which is a water-swellable, but water insoluble, fibrous cross-linked carboxy-functional polymer, said polymer containing (a) a plurality of repeating units of which at least about 80% contain at least one carboxyl functionality, and (b) about 0.05 to 1.5% cross-linking agent substantially free from polyalkenyl polyether, said percentages being based upon the weights of unpolymerised repeating unit and cross-linking agent, respectively, with (2) water, in the presence of paroxetine. The amount of calcium polycarbophil present is from about 0.1 to 99% by weight, for example about 10%. The amount of active agent present is from about 0.0001 to 65% by weight, for example between about 5 and 20%. The amount of water present is from about 5 to 200% by weight, for example between about 5 and 10%. The interaction is carried out at a pH of between about 3 and 10, for example about 6 to 7. The calcium polycarbophil is originally present in the form of a calcium salt containing from about 5 to 25% calcium.

Further particularly preferred formulations are described in US Patent No. 5,422,123.

Thus, a further preferred formulation of the invention comprises a system for the controlled release of paroxetine, comprising (a) a deposit-core comprising an effective amount of the active substance and having defined geometric form, and (b) a support-platform applied to said deposit-core, wherein said deposit-core contains at least the active substance, and at least one member selected from the group consisting of (1) a polymeric material which swells on contact with water or aqueous liquids and a gellable polymeric material wherein the ratio of the said swellable polymeric material to said gellable polymeric material is in the range 1:9 to 9:1, and (2) a single polymeric material having both swelling and gelling properties, and wherein the support-platform is an elastic support, applied to said deposit-core so that it partially covers the surface of the deposit-core and follows changes due to hydration of the deposit-core and is slowly soluble and/or slowly gellable in aqueous fluids. The support-platform may comprise polymers such as hydroxypropylmethylcellulose, plasticizers such as a glyceride, binders such as polyvinylpyrrolidone, hydrophilic agents such as lactose and silica, and/or hydrophobic agents such as magnesium stearate and glycerides. The polymer(s) typically make up 30 to 90% by weight of the support-platform, for example about 35 to 40%. Plasticizer may make up at least 2% by weight of the support-platform, for example about 15 to 20%. Binder(s), hydrophilic agent(s) and hydrophobic agent(s) typically total up to about 50% by weight of the support-platform, for example about 40 to 50%.

Paroxetine used in the present invention is suitably in the form of the free base or a pharmaceutically acceptable salt thereof. Preferably, paroxetine is suitably in the form of the hydrochloride hemihydrate.

Paroxetine hydrochloride hemihydrate may be prepared according to the procedures generally outlined in US Patent 4,721,723..

Paroxetine in the form of a controlled release and delayed release formulation can be used to treat and prevent the following disorders:
Alcoholism
Anxiety
Depression
Obsessive Compulsive Disorder
Panic Disorder
Chronic Pain
Obesity
Senile Dementia
Migraine
Bulimia
Anorexia
Social Phobia
Pre-Menstrual Syndrome (PMS)
Adolescent Depression
Trichotillomania
Dysthymia
Substance Abuse

These disorders are herein after referred to as "the disorders".

The present invention further provides the use of a controlled release and delayed release formulation containing paroxetine or a pharmaceutically acceptable salt thereof in the manufacture of a medicament, for treating and/or preventing the disorders.

The present invention also provides a pharmaceutical composition for use in the treatment and/or prevention of the disorders which comprises a controlled release and delayed release formulation containing paroxetine or a pharmaceutically acceptable salt thereof.

The following examples illustrate the present invention.

### Example 1 (Hydrophilic Matrix)

| Intragranular | % w/w |
|---|---|
| Paroxetine Hydrochloride | 11.45 |
| Methocel E5 | 1.25 |
| Lactose | 12.3 |

| Extragranular | |
|---|---|
| Methocel K100LV | 30.0 |
| Lactose | 44.0 |
| Magnesium Stearate | 1.0 |
| TOTAL | 100.0 |

### Example 2 (Hydrophilic Matrix)

| Intragranular | % w/w |
|---|---|
| Paroxetine Hydrochloride | 11.45 |
| Methocel ES | 1.25 |
| Lactose | 12.3 |

| Extragranular | |
|---|---|
| Methocel K100LV | 27.5 |
| Methocel K4M | 7.5 |
| Lactose | 39.0 |
| Magnesium Stearate | 1.0 |
| TOTAL | 100.0 |

### Example 3 (pH Sensitive Coat on Immediate Release Core)

| Tablet Core | %w/w |
|---|---|
| Paroxetine Hydrochloride | 11.45 |
| Lactose | 64.05 |
| Microcrystalline Cellulose | 20.0 |
| Sodium Starch Glycollate | 4.0 |
| Magnesium Stearate | 0.5 |
| TOTAL | 100.0 |

| Tablet Coating (apply approximately 6-10% of tablet core weight) | %w/w |
|---|---|
| Hydroxypropylmethylcellulose Phthalate | 90.0 |
| Triacetin | 10.0 |

### Example 4 (pH Sensitive Coat on Immediate Release Core)

Tablet Core as in Example 3

| Tablet Coating (apply approximately 6-10% of tablet core weight) | %w/w |
|---|---|
| Cellulose Acetate Phthalate | 90.0 |
| Diethyl Phthalate | 10.0 |

### Example 5 (Controlled Release Coating on Immediate Release Core)

Tablet Core as in Example 3

| Tablet Coating (apply approximately 5-12% of tablet core weight) | %w/w |
|---|---|
| Eudragit RS 100 | 86.0 |
| Dibutyl Phthalate | 10.0 |
| Talc | 4.0 |
| FD&C Yellow No. 6 | 0.01 |

### Example 6 (pH Sensitive Coat on Controlled Release Core.)

Tablet Core as in Example 3

Tablet Coating as in Example 3

### Example 7 (Encapsulated Controlled Release Coated Beads)

| Pellet | %w/w (approx) |
|---|---|
| Non Pareil Seed | 30 |
| Paroxetine Hydrochloride | 40 |
| Gelatin | 8 |
| Lactose | 20 |
| Talc | 2 |

| Coating | %w/w |
|---|---|
| Glycerylmonostearate | 36.6 |
| Glyceryldistearate | 53.4 |
| White Wax | 10.0 |

### Example 8 (Controlled release bilayer tablet)

| **Active Layer** | | |
|---|---|---|
| **Component** | **mg/tablet** | **Function** |
| Paroxetine Hydrochloride | 22.89* | Active |
| Methocel K4M | 15.00 | Hydrogel polymer |
| Lactose monohydrate | 62.0 | Hydrophilic agent |
| Polyvinylpyrrolidone | 3.0 | Binder |
| Magnesium stearate | 1.0 | Hydrophobic agent |
| Syloid 244 | 1.0 | Hydrophilic agent |

| **Support platform** | | |
|---|---|---|
| **Component** | **mg/tablet** | **Function** |
| Compritol 888 | 15.04 | Plasticizer |
| Lactose monohydrate | 29.32 | Hydrophilic agent |
| Polyvinylpyrrolidone | 4.0 | Binder |
| Magnesium stearate | 1.52 | Hydrophobic agent |
| Methocel E5 | 29.32 | Hydrogel polymer |
| Iron oxide | 0.08 | Colourant |
| Total tablet weight | 184.89mg | |

| | | |
|---|---|---|
| *Equivalent to 20mg paroxetine as free base. | | |

The powder blend for each layer was wet granulated in a high shear mixer/granulator and dried in a fluid bed drier. The bilayer tablets were compressed on a Manesty triple layer press.

### Example 9 (Enteric coated calcium polycarbophil formulation)

| **Core** | | |
|---|---|---|
| **Component** | **mg/tablet** | **Function** |
| Paroxetine Hydrochloride | 22.89* | Active |
| Calcium polycarbophil | 20.00 | Matrix |
| Lactose anhydrous | 146.11 | Hydrophilic agent/diluent |
| Polyvinylpyrrolidone | 10.0 | Binder |
| Magnesium stearate | 1.0 | Hydrophobic agent/lubricant |
| Water** | 0.024 | Granulating liquid |

| **Enteric coat** | | |
|---|---|---|
| **Component** | **mg/tablet** | **Function** |
| Eudragit | 22.19 | Polymer |
| Talc | 1.53 | Lubricant |
| Triethyl citrate | 1.00 | Plasticizer |
| Water** | 24.6 | Diluent |

| **Film coat** | | |
|---|---|---|
| Opadry pink | 10.5 | Film coat |
| Water** | 94.5 | Diluent |

| **Polish coat** | | |
|---|---|---|
| Opadry clear | 0.750 | |
| Water** | 29.3 | Diluent |

| | | |
|---|---|---|
| *Equivalent to 20mg paroxetine as free base. | | |
| **Removed during processing. | | |

The core constituents were wet granulated in a high shear mixer/granulator, and dried in a fluid bed drier. The magnesium stearate was then added and the mixture processed in a low shear mixer. The mix was then compressed on a B type rotary tablet press. Coating was carried out using an Accela cota.

### Example 10 (Controlled release bilayer tablet)

| **Active Layer** | | |
|---|---|---|
| **Component** | **mg/tablet** | **Function** |
| Paroxetine Hydrochloride | 22.89* | Active |
| Methocel K4M | 20.00 | Hydrogel polymer |
| Lactose monohydrate | 60.0 | Hydrophilic agent |
| Polyvinylpyrrolidone | 5.0 | Binder |
| Magnesium stearate | 1.0 | Hydrophobic agent |
| Syloid 244 | 1.0 | Hydrophilic agent |

| **Support platform** | | |
|---|---|---|
| **Component** | **mg/tablet** | **Function** |
| Compritol 888 | 14.72 | Plasticizer |
| Lactose monohydrate | 30.60 | Hydrophilic agent |
| Polyvinylpyrrolidone | 2.80 | Binder |
| Magnesium stearate | 0.80 | Hydrophobic agent |
| Methocel E5 | 30.60 | Hydrogel polymer |
| Syloid 244 | 0.40 | Hydrophilic agent |
| Iron oxide | 0.08 | Colourant |
| Total tablet weight | 189.89mg | |

| | | |
|---|---|---|
| *Equivalent to 20mg paroxetine as free base. | | |

The process was as described in Example 8.

### Example 11 (Controlled release bilayer tablet)

| **Active Layer** | | |
|---|---|---|
| **Component** | **mg/tablet** | **Function** |
| Paroxetine Hydrochloride | 22.89* | Active |
| Methocel K4M | 15.00 | Hydrogel polymer |
| Lactose monohydrate | 63.31 | Hydrophilic agent |
| Polyvinylpyrrolidone | 2.0 | Binder |
| Magnesium stearate | 1.0 | Hydrophobic agent |
| Syloid 244 | 0.40 | Hydrophilic agent |
| Support platform - as in Example 10. | | |
| Total tablet weight | 184.60mg | |

| | | |
|---|---|---|
| *Equivalent to 20mg paroxetine as free base. | | |

The process was as described in Example 8.

### Example 12 (Enteric coated controlled release bilayer tablet)

| **Active Layer** | | |
|---|---|---|
| **Component** | **mg/tablet** | **Function** |
| Paroxetine Hydrochloride | 28.61* | Active |
| Methocel K4M | 18.75 | Hydrogel polymer |
| Lactose monohydrate | 79.14 | Hydrophilic agent |
| Polyvinylpyrrolidone | 2.50 | Binder |
| Magnesium stearate | 1.25 | Hydrophobic agent |
| Syloid 244 | 0.50 | Hydrophilic agent |

| **Support platform** | | |
|---|---|---|
| **Component** | **mg/tablet** | **Function** |
| Compritol 888 | 15.04 | Plasticizer |
| Lactose monohydrate | 30.50 | Hydrophilic agent |
| Polyvinylpyrrolidone | 4.00 | Binder |
| Magnesium stearate | 0.80 | Hydrophobic agent |
| Methocel E5 | 29.32 | Hydrogel polymer |
| Syloid 244 | 0.32 | Hydrophilic agent |
| Iron oxide | 0.02 | Colourant |

| **Enteric coating** | | |
|---|---|---|
| **Component** | **mg/tablet** | **Function** |
| Eudragit | 13.27 | Polymer |
| Talc | 3.31 | Lubricant |
| Triethyl citrate | 1.33 | Plasticizer |
| Water** | 36.25 | Diluent |
| Total tablet weight | 228.66mg | |

| | | |
|---|---|---|
| *Equivalent to 25mg paroxetine as free base. | | |
| **Removed during processing. | | |

The process was as described in Example 9.

### Example 13

### GI tolerance study

The design of the study is outlined below
- Subjects:: Normal healthy volunteers
- Design:: Parallel group, placebo controlled, double blind
- Treatment:: (a) Placebo, (b) Immediate release paroxetine, (c)Example 8 formulation, (d) Example 8 formulation with enteric coating.
- Dosage:: 30 mg once daily for 3 days
- Number of subjects:: 452 evaluable (488 randomised, 485 evaluable)

The study was conducted to compare the incidence, severity and duration of nausea and vomiting, and diarrhoea (theoretically if the controlled release formulations slow down absorption of paroxetine then, as paroxetine is known to be prokinetic to the GI tract there may be an increased incidence).

Adverse experiences (AE) information was assessed each morning at the time of dosing and again 24 hours following the last dose. Investigators and subjects were given diary cards detailing how to classify severity of AEs in order to standardise as much as possible across all 6 centres.

Of the 485 evaluable subjects, 18 (3.7%) withdrew, 17 because of adverse events. Subjects with nausea/vomiting on the day of withdrawal were more common on (b) than either of (c) and (d).

The incidence of nausea/vomiting and diarrhoea is shown in the table below:

| | **(b)** | **(c)** | **(d)** | **Placebo** |
|---|---|---|---|---|
| **Incidence of nausea** | **59%** | **49%** | **39%** | **13%** |
| **Incidence of diarrhoea** | **15%** | **21%** | **20%** | **7%** |

The incidence of nausea was increased for both (b) and placebo compared to the expected rates of approximately 25% and 5% respectively for volunteers at these dosages for 3 days duration. The overall incidence of nausea was less on (c) and (d) than on (b). The severity of nausea was also decreased as shown in the next table.

| **Nausea severity** | **(b)** | **(c)** | **(d)** | **Placebo** |
|---|---|---|---|---|
| **None** | **50 (41%)** | **63 (52%)** | **74 (61%)** | **104 (87%)** |
| **Mild** | **45 (37%)** | **40 (33%)** | **30 (25%)** | **16 (13%)** |
| **Moderate** | **21 (17%)** | **17 (14%)** | **15 (12%)** | **0 (0%)** |
| **Severe** | **6 (5%)** | **1 (1%)** | **3 (2%)** | **0 (0%)** |

Severity of diarrhoea is reported in the table below:

| **Severity of diarrhoea** | **(b)** | **(c)** | **(d)** | **Placebo** |
|---|---|---|---|---|
| **None** | **104 (85%)** | **95 (79%)** | **97 (80%)** | **112 (93%)** |
| **Mild** | **16 (13%)** | **16 (13%)** | **16 (13%)** | **8 (7%)** |
| **Moderate** | **1 (1%)** | **8 (7%)** | **9 (7%)** | **0 (0%)** |
| **Severe** | **1 (1%)** | **2 (2%)** | **0 (0%)** | **0 (0%)** |

In conclusion, there appears to be a trend for (c) to reduce the incidence of nausea and the dropout rate due to adverse events in comparison to (b), but analysis of the results was complicated by a statistically significant treatment-by-centre difference. (d) shows a halving in the dropout rate and a fall in incidence of nausea of 20% (a proportional fall of 33%). In addition there is a reduction in severity of nausea of those individuals who report nausea on (c) and (d). There is an increase in incidence of diarrhoea on both of (c) and (d) in relation to (b), but this is confined to an increase in the number of individuals reporting moderate diarrhoea and there is no increase in those with severe diarrhoea.

## Claims

1. A controlled release and delayed release formulation which is adapted or intended for oral administration and which contains paroxetine or a pharmaceutically acceptable salt thereof.

2. A formulation according to claim 1 or 2, which comprises enteric coated tablets or caplets, wax or polymer coated tablets or caplets or time-release matrices, or combinations thereof.

3. A formulation according to claim 1 or 2, which is a polymeric controlled release composition comprising a reaction complex formed by the interaction of (1) a calcium polycarbophil component which is a water-swellable, but water insoluble, fibrous cross-linked carboxy-functional polymer, said polymer containing (a) a plurality of repeating units of which at least about 80% contain at least one carboxyl functionality, and (b) about 0.05 to 1.5% cross-linking agent substantially free from polyalkenyl polyether, said percentages being based upon the weights of unpolymerised repeating unit and cross-linking agent, respectively, with (2) water, in the presence of paroxetine or a pharmaceutically acceptable salt thereof.

4. A formulation according to claim 1 to 2, which is a system for the controlled release of paroxetine or a pharmaceutically acceptable salt thereof, comprising (a) a deposit-core comprising an effective amount of the active substance and having defined geometric form, and (b) a support-platform applied to said deposit-core, wherein said deposit-core contains at least the active substance, and at least one member selected from the group consisting of (1) a polymeric material which swells on contact with water or aqueous liquids and a gellable polymeric material wherein the ratio of the said swellable polymeric material to said gellable polymeric material is in the range 1:9 to 9:1, and (2) a single polymeric material having both swelling and gelling properties, and wherein the support-platform is an elastic support, applied to said deposit-core so that it partially covers the surface of the deposit-core and follows changes due to hydration of the deposit-core and is slowly soluble and/or slowly gellable in aqueous fluids.

5. Use of a controlled release and delayed release formulation according to any one of claims 1 to 4 in the manufacture of a medicament, for the treatement and/or prevention of alcoholism, anxiety, depression, obsessive compulsive disorder, panic disorder, chronic pain, obesity, senile dementia, migraine, bulimia, anorexia, social phobia, pre-menstrual syndrome (PMS), adolescent depression, trichrotillomania, dysthymia or substance abuse.

6. A process for the preparation of a formulation according to any one of claims 1 to 4, which comprises combining the constituents thereof in the required proportions.

## Patentansprüche

1. Zubereitung zur gesteuerten Freisetzung und verzögerten Freisetzung, die für orale Verabreichung angepasst oder bestimmt ist und die Paroxetin oder ein pharmazeutisch verträgliches Salz davon enthält.

2. Zubereitung nach Anspruch 1, die magensaftresistant überzogene Tabletten oder Caplets, Wachs oder Polymer überzogene Tabletten oder Caplets oder Matrizen zur zeitlich gesteuerten Abgabe oder deren Kombinationen umfasst.

3. Zubereitung nach Anspruch 1 oder 2, die eine polymere Zusammensetzung zur gesteuerten Freisetzung ist, umfassend einen Reaktionskomplex, der durch die Wechselwirkung eines (1) Polycarbophil-Calcium Bestandteils, der ein in Wasser quellbares, aber wasserunlösliches, faserförmiges, vernetztes, Carboxy-funktionalisiertes Polymer ist, wobei das Polymer (a) eine Vielzahl an sich wiederholenden Einheiten, von denen mindestens etwa 80 % mindestens eine Carboxylfunktionalität enthalten, und (b) etwa 0,05 bis 1,5 % Vernetzungsmittel, das im wesentlichen frei von Polyalkenylpolyether ist, enthält, wobei die Prozentgehalte sich jeweils auf die Gewichte der nicht polymerisierten sich wiederholenden Einheiten und des Vernetzungsmittels beziehen, mit (2) Wasser in Gegenwart von Paroxetin oder einem pharmazeutisch verträglichen Salz davon gebildet wird.

4. Zubereitung nach Anspruch 1 oder 2, die ein System zur gesteuerten Freisetzung von Paroxetin oder eines pharmazeutisch verträglichen Salzes davon ist, umfassend (a) einen Abscheidungskem, der eine wirksame Menge des Wirkstoffs umfasst und eine definierte geometrische Form hat, und (b) ein Stützaufbau, der auf den Abscheidungskern aufgetragen ist, wobei der Abscheidungskern mindestens den Wirkstoff und mindestens einen aus (1) einem polymeren Material, das bei Berührung mit Wasser oder wässrigen Flüssigkeiten quillt, und einem gelierbaren polymeren Material, wobei das Verhältnis von quellbarem polymeren Material zu gelierbarem polymeren Material im Bereich von 1:9 bis 9:1 liegt und (2) einem einzelnen polymeren Material, das sowohl Quell- als auch Geliereigenschaften hat, ausgewählten Bestandteil enthält und wobei der Stützaufbau ein elastischer Träger ist, der so auf den Abscheidungskern aufgetragen ist, dass er die Oberfläche des Abscheidungskems teilweise bedeckt und Veränderungen auf Grund von Hydration des Abscheidungskems folgt, und langsam löslich und/oder langsam gelierbar in wässrigen Flüssigkeiten ist.

5. Verwendung einer Zubereitung zur gesteuerten Freisetzung und verzögerten Freisetzung nach einem der Ansprüche 1 bis 4 zur Herstellung eines Medikaments zur Behandlung und/oder Vorbeugung von Alkoholismus, Angstzuständen, Depression, zwanghafter Persönlichkeitsstörung, Panikattacke, chronischem Schmerz, Obesität, seniler Demenz, Migräne, Bulimie, Anorexie, Sozialphobie, prämenstruellem Syndrom (PMS), Depression bei Heranwachsenden, Trichotillomanie, Dysthemie oder Suchtmittelmissbrauch.

6. Verfahren zur Herstellung einer Zubereitung nach einem der Ansprüche 1 bis 4, das das Zusammenfügen der Bestandteile in den benötigten Verhältnissen umfasst.

## Revendications

1. Formulation à libération contrôlée et à libération retardée qui est apte ou destinée à l'administration orale et qui contient de la paroxétine ou un de ses sels pharmaceutiquement acceptables.

2. Formulation suivant la revendication 1, qui comprend des comprimés ou caplets à enrobage entérique, des comprimés ou caplets enrobés d'une cire ou d'un polymère ou des matrices à libération progressive, ou leurs associations.

3. Formulation suivant la revendication 1 ou 2, qui est une composition polymère à libération contrôlée comprenant un complexe réactionnel formé par interaction (1) d'un constituant polycarbophile calcique qui est un polymère à fonctionnalité carboxy réticulé fibreux apte au gonflement dans l'eau, mais insoluble dans l'eau, ledit polymère contenant (a) une pluralité de motifs répétés dont au moins environ 80 % contiennent au moins une fonctionnalité carboxyle, et (b) environ 0,05 à 1,5 % d'un agent de réticulation pratiquement dépourvu de polyalcénylpolyéther, lesdits pourcentages étant basés sur les poids de motif répété non polymérisé et d'agent de réticulation, respectivement, avec (2) de l'eau, en présence de paroxétine ou d'un de ses sels pharmaceutiquement acceptables.

4. Formulation suivant la revendication 1 ou 2, qui est un système pour la libération contrôlée de paroxétine ou d'un de ses sels pharmaceutiquement acceptables comprenant (a) un noyau à dépôt comprenant une quantité efficace de la substance active et ayant une forme géométrique définie et (b) une plate-forme de support appliquée audit noyau à dépôt, ledit noyau à dépôt contenant au moins la substance active, et au moins un membre choisi dans le groupe consistant en (1) une matière polymère qui gonfle au contact de l'eau ou de liquides aqueux et une matière polymère gélifiable, le rapport de ladite matière polymère pouvant gonfler à ladite matière polymère gélifiable étant compris dans l'intervalle de 1:9 à 9:1, et (2) une matière polymère unique ayant à la fois des propriétés de gonflement et de gélification, la plate-forme de support étant un support élastique, appliqué audit noyau à dépôt de telle sorte qu'il couvre partiellement la surface du noyau à dépôt et qu'il suive les variations dues à l'hydratation du noyau à dépôt et qu'il soit lentement soluble et/ou lentement gélifiable dans des fluides aqueux.

5. Utilisation d'une formulation à libération contrôlée et à libération retardée suivant l'une quelconque des revendications 1 à 4, dans la production d'un médicament destiné au traitement et/ou à la prévention de l'alcoolisme, de l'anxiété, de la dépression, du trouble obsessionnel compulsif, des troubles du type de la panique, de la douleur chronique, de l'obésité, de la démence sénile, de la migraine, de la boulimie, de l'anorexie, de la névrose phobique, du syndrome prémenstruel (PMS), de la dépression de l'adolescence, de la trichotillomanie, de la dysthymie ou de la pharmacodépendance.

6. Procédé pour la préparation d'une formulation suivant l'une quelconque des revendications 1 à 4, qui comprend l'association des constituants de cette formulation en les proportions requises.
